# EUROPEAN PATENT SPECIFICATION

(11) **EP 3 634 566 B1**
(45) Date of publication and mention of the grant of the patent: **06.10.2021**
(21) Application number: 18726039.3
(22) Date of filing: 07.05.2018
(51) Int. Cl.: A61N 1/04

(54) **COMPRESSION GARMENT FOR PROVIDING NEUROMUSCULAR ELECTRICAL STIMULATION**
KOMPRESSIONSBEKLEIDUNG FÜR NEUROMUSKULÄRE ELEKTRISCHE STIMULATION
VÊTEMENT DE COMPRESSION POUR LA STIMULATION ÉLECTRIQUE NEUROMUSCULAIRE

(30) Priority: 08.05.2017 DK PA201770323
(43) Date of publication of application: 15.04.2020
(73) Proprietor: Aarhus Universitet, 8000 Aarhus C (DK)
(72) Inventor: SUETTA, Charlotte Arneboe, 2000 Frederiksberg (DK); FRANDSEN, Ulrik, deceased (DK); NYGAARD, Jens Vinge, 8530 Hjortshøj (DK); MIKKELSEN, Peter Høgh, 8260 Viby J (DK)
(74) Representative: Plougmann Vingtoft a/s
(86) International application number: PCT/DK2018/050098
(87) International publication number: WO 2018/206067

(56) References cited:
- WO-A1-2016/038545
- US-A1- 2004 030 270
- US-A1- 2004 254 624
- US-A1- 2007 106 343
- US-A1- 2014 206 947
- US-A1- 2014 213 940
- US-A1- 2015 202 351
- US-A1- 2015 224 011
- US-A1- 2016 303 363
- US-A1- 2017 056 644

## Description

### Technical field of the invention

The present invention relates to a compression garment comprising electrical stimulation means.

### Background of the invention

Skeletal muscle tissue accounts for about 40% of the human body mass and, in addition to its power-generating role, skeletal muscle represent a crucial factor in maintaining homeostasis of glucose metabolism and as an energy reservoir in catabolic conditions. Given its central part in human mobility and metabolic function, any deterioration in the contractile and metabolic properties of skeletal muscle has a significant effect on human health.

With immobilization loss of muscle mass starts early and fast. Significant changes in gene expression levels of genes regulating muscle atrophy can be observed within the first two days of muscle disuse and significant muscle fiber atrophy is evident after only four days of lower limb immobilization. On the other hand, it takes much longer to regain the loss of muscle, despite intensive exercise regimens, especially in older individuals.

Therefore, it seems paramount to counteract loss of muscle mass and muscle strength as early as possible after patients are immobilized. It has been demonstrated that early exercise training in postoperative patients as well as in critically ill patients receiving mechanical ventilation is feasible, safe, and beneficial for respiratory and lower limb muscles.

Despite this knowledge, an infinite number of medical and surgical illness states still lead to the development of hospital associated. Although much effort is done to minimize surgical intervention, effects of major surgery are still associated with an increased risk of morbidity, convalescence and disability.

Neuromuscular electrical stimulation (NMES) is a non-invasive method that stimulates the muscle without active participation and is known to counteract muscle atrophy during periods of inactivity/immobility. NMES has been used in the rehabilitation of sports injuries and as a means to prevent muscle wasting in immobilized and also in critically ill patients.

Neuromuscular electrical stimulation (NMES) is a treatment modality to evoke a muscle contraction through an electrical impulse via surface electrodes. For this technique, no patient cooperation is required and NMES has been identified as an alternative to active exercise in critically ill patients that are often under the influence of sedative drugs and therefore not able to collaborate in active voluntary muscle training. NMES is a well-established technology with documented results of the effect and with several vendors on the market. All conventional NMES systems includes stimulation pads patch for electrical energy transfer to biological tissue. The stimulation pad provides contact between the targeted biological tissue and the electrical probes providing the electrically stimulating current. The purpose of such stimulation pad is thus to impose electric signals onto the human body.

US2017/056644 A1 discloses a mixed layer textile product e.g. garment, for user, having conductive fibers interlaced with another fibers extending lateral to pathway, and conductive segment comprising electrical resistance differing from another electrical resistance.

However, *despite* the positive effects on preserving skeletal muscle during bed rest and periods immobility, NMES is not widely used neither in the rehabilitation of hospitalized patients, nor in the post-operative rehabilitation in other settings. Deep venous thrombosis (DVT) is a common late complication among a variety of post- operative patients or for other reasons immobilized patients, e.g. geriatric, orthopedic and neurological patients, apoplexies in both acute and late treatment regimens on cerebral palsy, paraplegics (acute and long term rehabilitation) and patients at intensive care units. A part from muscle atrophy, immobilization markedly increases the risk of deep venous thrombosis (DVT) and edema.

Elastic compression stockings is part of the clinical treatment regime for a wide array of patients within the geriatric, orthopedic, neurological and rheumatologic field. Accordingly, it is an efficient treatment to venous insufficiency and is widely used to patients at risk of developing DVT or already suffering from one, Elastic compression stockings are also effective to treat edema in lower and upper limbs. Hence, an improved NMES system would be advantageous, and in particular a more efficient and/or reliable NMES system would be advantageous to get it implemented in the rehabilitation of hospitalized patients, and in the post-operative rehabilitation in other settings.

### Summary of the invention

Main obstacles for why NMES systems is not widely implemented may be that
1) Current stimulation pads are not developed to be attached to the skin for more than 1 hour. Current NMES systems cannot be left on the skin over longer periods (>1 hour) because they do rapidly lead to rash or irritation of the skin and therefore the pads must be taken off the skin of the user after every single incident of NMES treatment. This is time consuming for the health personnel.
2) A positive effect of a NMES system is based on optimal contact between stimulation pad and the skin; otherwise the electrical stimulus will be diffuse and the electrical stimulus will be confined to the upper skin layer instead of being transferred to the muscle. This will incur pain to the patient. Because of such pain, patients are often disinclined to accept NEMS treatment. In currently available NMES systems, the contact between the stimulation pad and the skin declines over time and thereby the effect of NMES declines with prolonged usage and the discomfort of pain imposed to the patient will increase. One solution to this problem is the use of contact gels.
3) Current used NMES programs are time limited (e.g. 30-60 min 3-5 times per week) partly to avoid irritation of the skin and partly due to the logistic challenges explained herein.
4) Patients wearing compression stockings will have to have the stockings removed before the electrodes can be positioned. Similar the electrodes must be removed and the stockings placed again after the stimulation. Again this is time consuming. Thus, there is a great need of a compression garment, with integrated electrodes so the garment would not have to be removed before applying NMES, which would make the workflow of the physiotherapist markedly easier and thereby increasing the compliance.

Thus, it is an object to provide a stimulation system which:
∘ Will not cause rash or skin irritation even when being placed on the skin for several days and may thus be left on the skin and repeatedly used for multiple incidents of NMES treatment. This will improve the workflow for health personnel because the application and removal several times per day is thereby no longer necessary and thereby the need for supervision by health personnel in the process of electric stimulation and treatment is reduced.
∘ Will enable optimal skin-electrode contact for transfer of electrical energy when being placed on the skin for several days.
∘ Will make it possible to stimulate more frequently because it may be left on the skin for several days. This will expectedly lead to improved effect of the NMES treatment.
∘ Does not require the use of contact gel.
∘ Which may comprise a data storage and feedback system during long term use of the NMES system.

In summary, the present invention relates to a compression garment, preferably a compression stocking, for providing neuromuscular electrical stimulation (NMES), the compression garment comprises at least two integrated electrode pads enabling NMES, wherein the integrated electrode pads comprise silicone polymers, the silicone polymer further comprising graphene, the at least two pads being adapted to be in contact with the skin when in use. The compression garment further comprises connection means for electrically connecting the integrated electrode pads to a stimulation device, the stimulation device being adapted to run one or more neuromuscular electrical stimulation patterns through the integrated electrode pads. The invention further relates to a system comprising the compression garment.

Thus, one aspect of the invention relates to a compression garment (2), preferably a compression stocking, for providing neuromuscular electrical stimulation (NMES), the compression garment comprising:
- at least two integrated electrode pads (3) adapted to provide NMES, wherein the electrode pads comprise silicone polymers, the silicone polymer further comprises graphene, the at least two electrode pads being adapted to be in contact with the skin of a subject when in use; and
- connection means for electrically connecting the at least two integrated electrode pads (3) to an associated stimulation device (4), the stimulation device being adapted to implement one, or more, neuromuscular electrical stimulation patterns through the integrated electrode pads (3) when in contact with the skin.

Example 5 shows that none of the three test-subjects experienced any discomfort, rash or pain during a 7 days test trial. This was in contrast to commercially available closed pad and pads with gel.

Another aspect of the present invention relates to a neuromuscular electrical stimulation system (1) comprising
- a compression garment (2), preferably a compression stocking, with at least two integrated electrode pads (3) adapted to provide neuromuscular electrical stimulation (NMES), wherein the at least two integrated electrode pads (3) comprise silicone polymers, the silicone polymer further comprises graphene, the at least two electrode pads being adapted to be in contact with the skin of a subject when in use; and
- a stimulation device (4) electrically connected to the at least two integrated electrode pads (3) in the compression stocking (2), adapted to implement one, or more, neuromuscular electrical stimulation patterns through the integrated electrode pads (3).

### Brief description of the figures

Figure 1 shows the system according to the invention. The picture shows a preferred embodiment of the invention where the tube shaped compression garment is a compression stocking. 1) system; 2) compression stocking; 3) integrated stimulation pads; 4) stimulation device.
Figure 2 shows an exploded schematic figure of an interwoven stimulation pad in a garment 2. A, B, C) are electrically conductive silicone fibers; G) indicates one or more microporous and hydrophobic layers; E) One or several connectors to the garment from which wiring ensure connectivity to each pad; D) indicates a distance between the silicone threads A, B, C. 5 indicates a cover layer.
Figure 3 shows the electrical resistance (Through Plane Resistance (W)) of silicone with different concentrations of graphene. X-axis: Wt % Graphene in silicone. Y-axis: Ohm.
Figure 4 shows show how water evaporate from a surface not covered, covered by a silicone mesh, and when a silicone mesh is impregnated by one or ten sprayed layers of PTFE. Thus the distance, D, between the silicone threads, the diameter of the threads, the integration into the woven textile, and the presence of PTFE determines overall permeability. X-axis: Time (min.). Y-axis: Water evaporation (g).
Figure 5 shows a figure where an occlusion device is shown. The occlusion device may be integrated with a neuromuscular electrical stimulation system according to the present invention.
Figure 6 is a photograph of a compression garment according to the present invention.
Figure 7 shows photos of the right lower limb of one subject each day during a 7 day test.
Figure 8 shows a model of a 3D printed pad according to the invention.

The present invention will now be described in more detail in the following.

### Detailed description of the invention

### Compression garment

In a general aspect the invention relates to a compression garment (2), preferably a compression stocking, for providing neuromuscular electrical stimulation (NMES), the compression garment comprising:
- at least two integrated electrode pads (3) adapted to provide NMES, wherein the electrode pads comprise one or more elastomers, preferably silicone, the elastomers further comprise electrically conductive carbon particles, preferably graphene, the at least two electrode pads being adapted to be in contact with the skin of a subject when in use; and
- connection means for electrically connecting the at least two integrated electrode pads (3) to an associated stimulation device (4), the stimulation device being adapted to implement one, or more, neuromuscular electrical stimulation patterns through the integrated electrode pads (3) when in contact with the skin.

In a first aspect the invention relates to a compression garment (2), preferably a compression stocking, for providing neuromuscular electrical stimulation (NMES), the compression garment comprising:
- at least two integrated electrode pads (3) adapted to provide NMES, wherein the electrode pads comprise silicone polymers, the silicone polymer further comprises graphene, the at least two electrode pads being adapted to be in contact with the skin of a subject when in use; and
- connection means for electrically connecting the at least two integrated electrode pads (3) to an associated stimulation device (4), the stimulation device being adapted to implement one, or more, neuromuscular electrical stimulation patterns through the integrated electrode pads (3) when in contact with the skin.

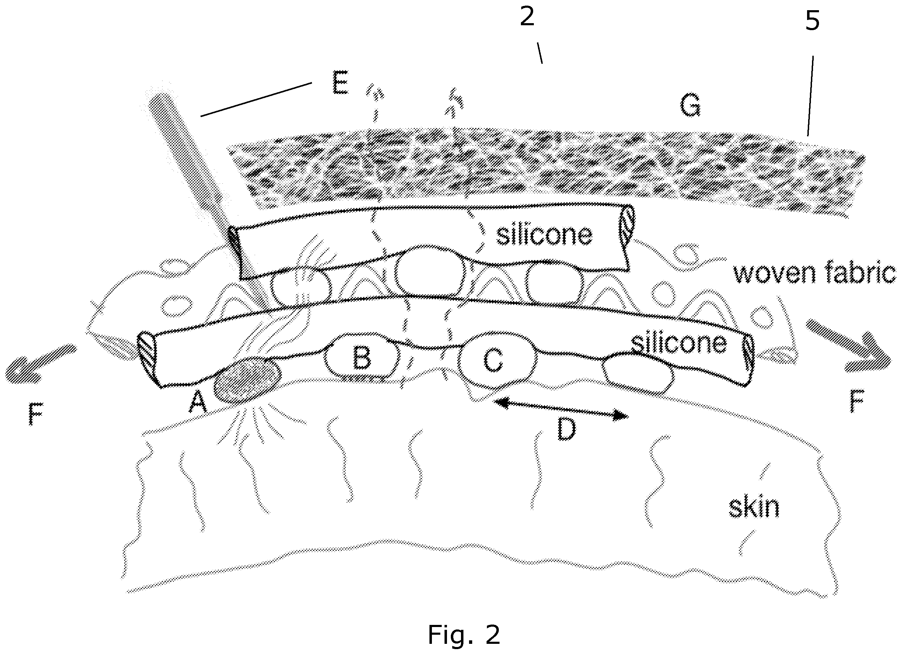

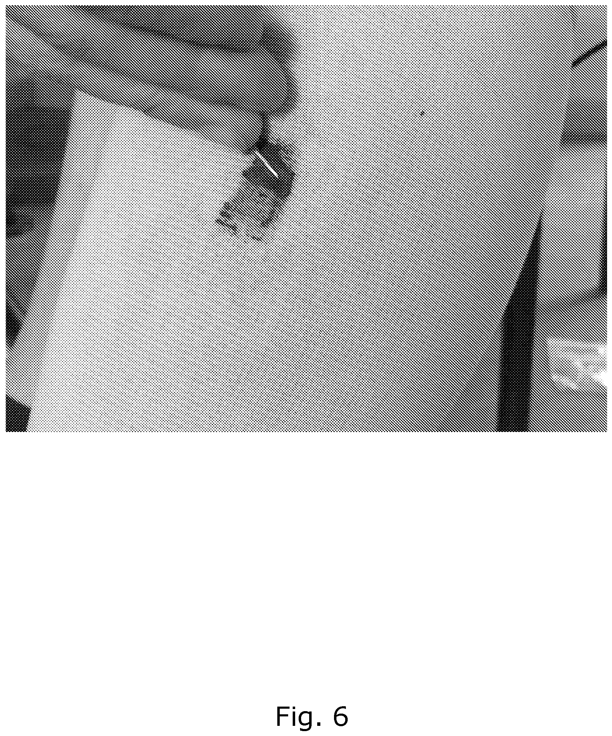

Figure 2 shows an exploded schematic figure of an interwoven stimulation pad in a garment 2. A, B, C) are electrically conductive silicone fibers; G) indicates one or more microporous and hydrophobic layers; E) One or several connectors to the garment from which wiring ensure connectivity to each pad; D) indicates a distance between the silicone threads A, B, C. Figure 6 is a photograph of a compression garment according to the present invention.

In a further embodiment, the elastomer is selected from the group consisting of polyacrylate, acrylic ester rubber, polyacrylonitrile, poly (acrylonitrile-co-butadiene-co-styrene), poly(acrylonitrileco-methyl methacrylate), polyamide, polyamideimide, polyester, polyether ether ketone, polyether ester, polyethylene, ethylene-propylene rubber, poly(ethylene-co-tetrafluoroethylene), poly(ethylene-co -vinyl acetate), poly(ethylene-co-vinyl alcohol), fluorosilicones, perfluoroalkoxy polymers, (natural) rubber, poly(methyl methacrylate-co-acrylonitrileco-butadiene-co-styrene), poly(methyl methacrylate-cobutadiene-co-styrene), nitriles, olefins, polyphosphazenes, polypropylene, poly(methyl methacrylate), polyurethanes, polyvinyl chloride, polyvinyl fluorides, latex (preferably medical grade latex) and silicones, preferably silicone, even more preferably medical grade silicone.

In a further embodiment, the electrically conductive carbon particles are selected from the group consisting of carbon nanotubes, single-wall carbon nanotubes, multiwall carbon nanotubes, carbon nanohorns, carbon nanoonions, fullerenes, graphite, graphene, carbon fibers, carbon black and conductive carbon black, preferably graphene.

In an embodiment, the elastomers comprise 10-40% (w/w) electrically conductive carbon particles and 60-90% (w/w) elastomers, preferably 20-35% electrically conductive carbon particles and 65-80% elastomers, and more preferably 23-32% electrically conductive carbon particles and 68-77% elastomers.

In a preferred embodiment, the silicone polymers comprises 10-40% (w/w) graphene and 60-90% (w/w) silicone, preferably 20-35% graphene and 65-80% silicone, and more preferably 23-32% graphene and 68-77% silicone. Example 3 shows optimization of the silicone: graphene ratio.

In another embodiment, the graphene (or electrically conductive carbon particles) have an average particle size below 10 microns, preferably below 20 microns, or more preferably below 30 microns.

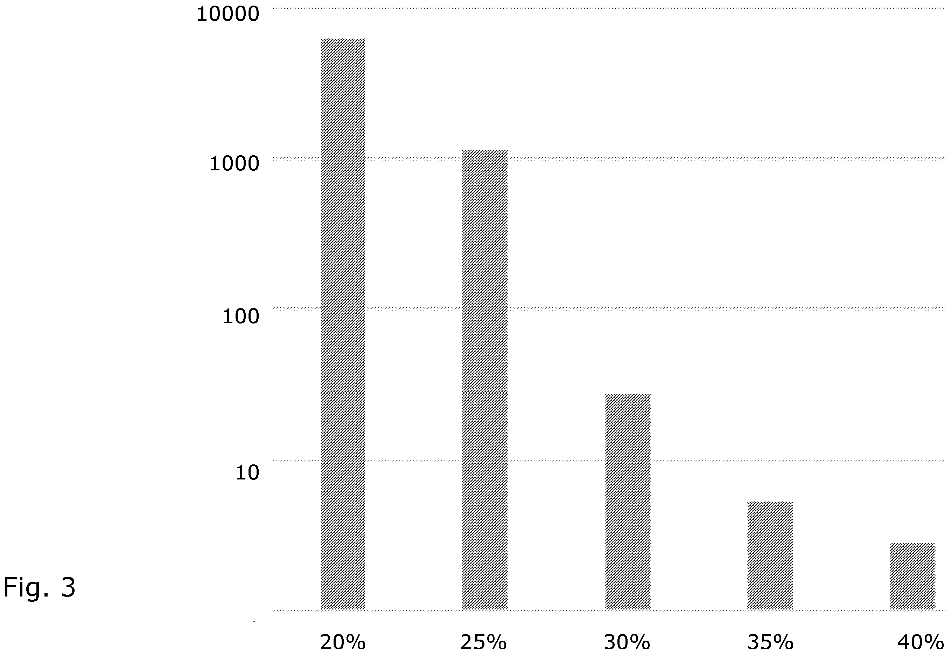

In yet an embodiment, the silicone polymers (or elastomers) comprising graphene (or electrically conductive carbon particles), has an electrical impedance of less than 300 ohms, such as in the range 3-300 ohms, such as in the range 5-100 ohms, such as in the range 10-80 ohms. Figure 3 shows the electrical resistance of silicone with different concentrations of graphene.

In a further embodiment, the integrated electrode pads (3) comprise silicone polymer strands (or elastomers) positioned with an average distance between the individual polymer strands of 0 to 0.5 mm, such as 0.15 mm to 0.45 mm, preferably with an average distance of 0.3 to 0.4 mm. This micro-structuring of the pads improves evaporation from the skin and thus let the skin "breath". Example 5 shows the effect on skin over longer periods of time of different pads.

In an embodiment, the diameter of the silicone polymer strands (or elastomers) is from 10 to 250 micron, preferably 100 to 300 micron, more preferably around 200 micron.

In a preferred embodiment the diameter of the silicone polymer strands (or elastomers) is from 10 micron to 5 mm, such as from 200 micron to 5 mm, preferably such as from 1 mm to 5 mm, or such as from 2 mm to 5 mm.

In a preferred embodiment the silicone polymers (or elastomers) has a diameter in the range 0.1-0.3 mm and the distance between each polymer strand is in the range 0.3-0.5 mm. even more preferred the silicone polymers (or elastomers) have a diameter of (about) 0.2 mm and the distance between each polymer strand is (around) 0.4 mm. This specific embodiment will result in 17 polymer strands per cm² and the area of the polymers per cm² (=100 mm2) is 34 mm2 which results in that 34% of the area will be covered with the conductive polymers. This corresponds to covering between 66 and 130 pores per cm2, since there are typically 200 ― 400 glands / cm² of skin surface. The sweat glands are distributed almost all over the human body, in varying densities. Its water-based secretion represents a primary form of cooling in humans and features a natural regulation of the humidity at the interface between the electrical pad and the skin. The natural presence of gland-delivered water, lowers the electrical resistance over the skin.

In yet an embodiment, the distance between each polymer strand is in the range of 0.15 mm to 5 mm, such as 0.3 to 5 mm, preferably with an average distance of such as 1-5 mm, or such as 2-5 mm.

The silicone polymers (or elastomers) may have different shapes. In an embodiment, the compression garment according to claim 6 or 7, wherein the silicone polymer strands (or elastomers) in a cross-section view are substantially cylindrical, star-shaped or strings including circular formations or combinations thereof.

The effectiveness of the compression garment and in particular the electrical pads, may depend on the stiffness of the integrated silicone polymers (or elastomers). Thus, in an embodiment, the compression garment with the silicone polymers (or elastomers) comprising graphene (or electrically conductive carbon particles) has a stiffness in the range 0.05 to 0.3 MPa, preferably in the range from 0.01 to 0.02 MPa. The described ranges mimic the elasticity of the skin. It varies with the different location of the body from 0.050 MPa to 0.3 MPa. It is though difficult to reach such a low elasticity, because the addition of graphene increase the stiffness, and optimally around 27% of the volume needs to be carbon. However the open structure of the pad (from the lines of silicone (or elastomers)) lower the elasticity. Thus, the mentioned stiffness relates to the stiffness of the overall structured composite, and not the silicone with graphene alone. Thus, in a further embodiment the at least two integrated electrode pads are woven into the garment constituting the compression garment.

In yet an embodiment, the graphene has an average size and distribution within the silicone polymer so as to the enable electrical conduction through the at least two electrode pads to the skin of the subject.

The number of integrated electrode pads (3) in the compression garment may vary. Thus, in an embodiment the compression garment (2) comprises 2-10 integrated electrode pads (3), such as 4-8 integrated electrode pads, or such as 4-6 integrated electrode pads. The distance between the at least two electrode pads may also vary. Thus, in an embodiment, the at least two electrode pads are positioned with a distance of around 0.1 cm, 0.2 cm, 0.3 cm, 0.4 cm, 0.5 cm, 0.6 cm, 0.7 cm., 0.8 cm, 0.9 cm, 1.0 cm, 1.5, cm 2.0, 2.5 cm, or 3.0 cm. For larger body parts, e.g. leg muscle, the electrode pad distance could up to 10 cm, 15 cm, or 20 cm. In some embodiments, the electrode pads can be arranged in a plurality of clusters.

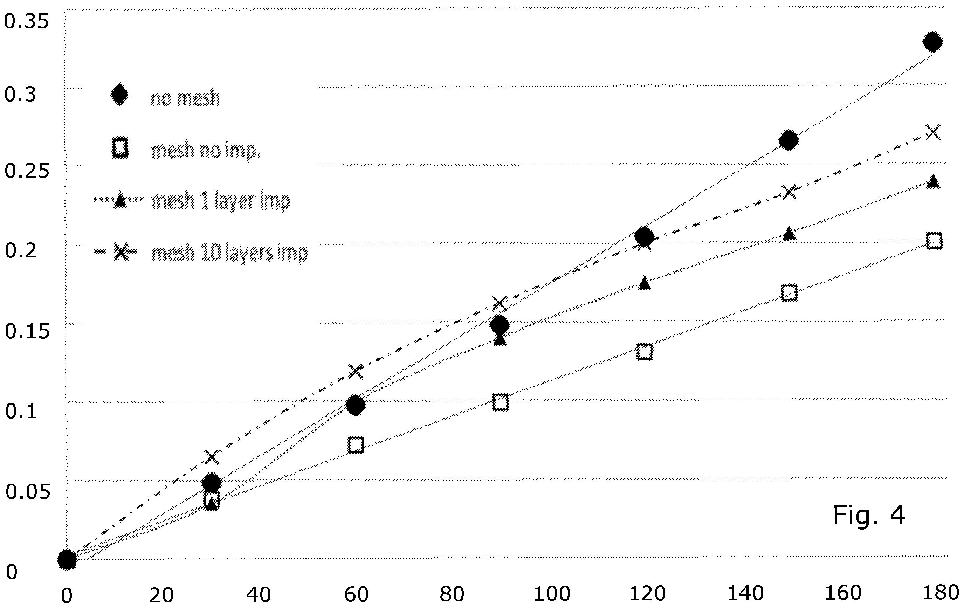

The pads may be further optimized to improve the conductivity of the pads. Thus, in an embodiment the part of the electrodes adapted to be in contact with the skin of the subject, is further covered with one or more, preferably hydrophobic, cover layers (5) on a side adapted to face away from the skin, to maintain moisture at the skin-electrode interface. In yet an embodiment the one or more cover layers (5) comprise polytetrafluoroethylene (PTFE), polyurethanes, polyethylenes, polypropylenes, polyethylene terephelate, and/or polyesters, preferably formulated into microporous fabrics. Figure 4 shows optimization data relating to cover layers. Thus, Figure 4 shows show how water evaporate from a surface not covered, covered by a silicone mesh, and when a silicone mesh is impregnated by one or ten sprayed layers of PTFE. Thus the distance, D, between the silicone threads, the diameter of the threads, the integration into the woven textile, and the presence of PTFE determines overall permeability.

The compression garment may of course to connected to a stimulation device. Thus, in an embodiment the associated stimulation device (4) is connected detachable to the at least two integrated electrode pads via one, or more, connectors (E).

The compression garment may comprise different materials. Thus, in yet an embodiment, the compression garment comprises polyamide polytetrafluoroethylene (PTFE), polyurethanes, polyethylenes, polypropylenes, polyethylene terephelate, and/or polyesters, fabrics and/or elastan, preferably such as comprising 50-96% soft polyamide and 4-30% elastan, more preferably such as 70-90% polyamide and 10-20% elastan.

The shape of the compression garment may also vary depending on the specific use. Thus, in a further embodiment the garment is tube-shaped or substantially cylindrical, preferably with a closed end such as in the form as a compression stocking. In a further embodiment, the integrated electrode pads cover an area of 5-50%, such as 10-50% or such as 20-50 % of the total surface area of the compression garment. In yet a further embodiment the compression garment has a graduated compression along the length of the garment with the highest compression at the most distant part of the extremity to be treated.

The integrated electrode pads may be adapted to provide electrical stimulation with different currents. Thus, in an embodiment, the integrated electrode pads are adapted to provide an electrical stimulation with peak currents up to 500mA, preferably 1000 mA, more preferably 1500 mA, preferably with a frequency of 10-60 Hz.

### Neuromuscular electrical stimulation system

The compression garment according to the invention may also form part of a neuromuscular electrical stimulation system. Thus in another aspect, the invention relates to a neuromuscular electrical stimulation system (1) comprising
- a compression garment (2), preferably a compression stocking, with at least two integrated electrode pads (3) adapted to provide neuromuscular electrical stimulation (NMES), wherein the at least two integrated electrode pads (3) comprise silicone polymers, the silicone polymer further comprises graphene, the at least two electrode pads being adapted to be in contact with the skin of a subject when in use; and
- a stimulation device (4) electrically connected to the at least two integrated electrode pads (3) in the compression stocking (2), adapted to implement one, or more, neuromuscular electrical stimulation patterns through the integrated electrode pads (3).

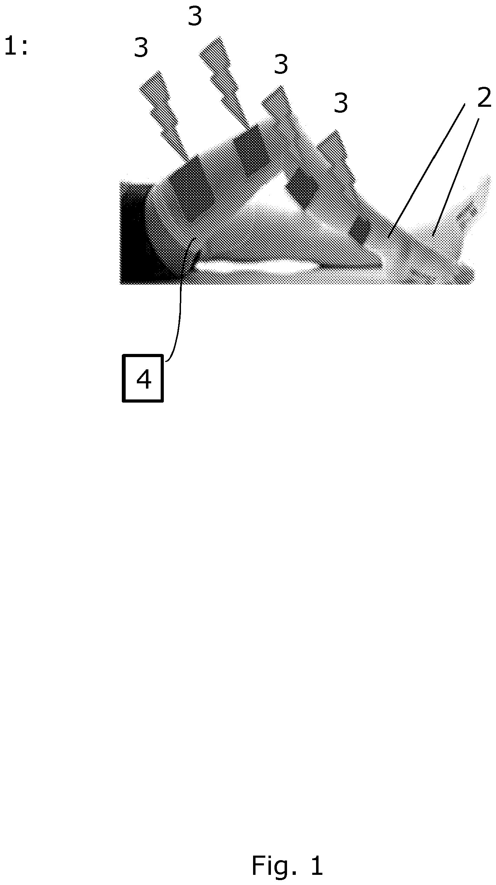

Figure 1 shows the system according to the invention. The picture shows a preferred embodiment of the invention where the tube shaped compression garment is a compression stocking. 1) system; 2) compression stocking; 3) integrated stimulation pads; 4) stimulation device.

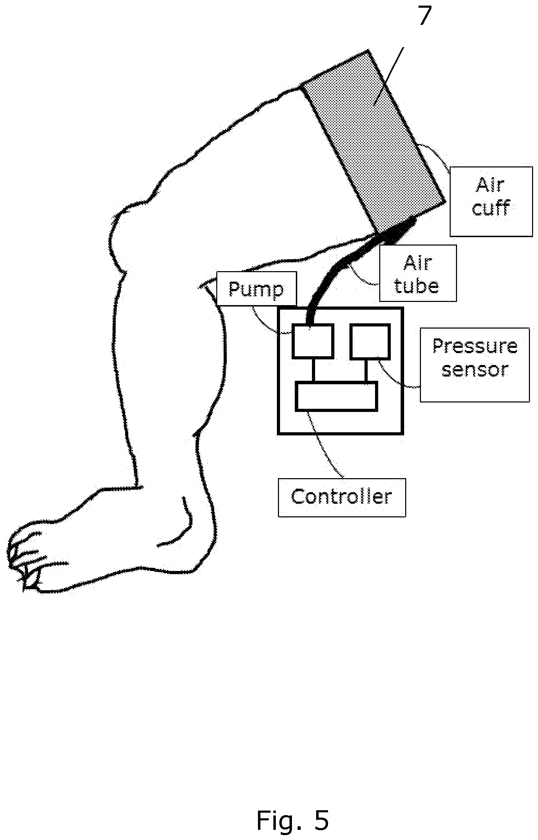

In an embodiment, the system further comprises a blood occlusion device (7) adapted to be positioned on a subject to reduce the blood flow to and/or from the muscle during at least part of the neuromuscular electrical stimulation. In yet an embodiment the blood occlusion device is adapted to provide a functional compression pressure in the range 50-150 mmHg, such as in the range preferably in the range 50-110 mmHg, or such as in the range 50-90 mmHg, preferably under the control of the stimulation device. In yet a further embodiment, the blood occlusion device is a pneumatic cuff. Figure 5 shows a figure where an occlusion device is shown. The occlusion device may be integrated with a neuromuscular electrical stimulation system according to the present invention. In another embodiment, the compression of the occlusion device is controllable by the stimulation device (4) and wherein the compression form part of the one or more neuromuscular electrical stimulation patterns. In example 2, examples of stimulation patterns are shown.

The invention will now be described in further details in the following non-limiting examples.

### Examples

### Example 1

### Occlusion

As occlusion must be supervised and monitored to avoid critical events during its application this is not build into the garment. An occlusion device is significant of size and its presence as a part of the garment design will introduce discomfort and risk of bedsores. Thus, as indicated in figure 5 occlusions are conducted by the addition of a second device, for instance over the upper leg of the patient.

### Example 2

### Example of a stimulation patterns

The included patient segments are specific examples of a variety of treatments according to a predetermined schedule. The following examples are given to provide knowledge on a number of the variations that NEMS protocols administered by PMPS can provide:
High intensity stimulation (muscle atrophy and reduced protein synthesis):
∘ The typical shape of pads are circular, elliptical, n-sided polygons with an area of 0.5 to 10 cm². The pads can be arranged in orthogonal, hexagonal, random or clustered patterns to provide optimum stimuli to the tissue. The distribution is dependent on the location on the body. The inter-pad distance can be down to 0.1 mm and up to several cm in the case of the application to a lower limb. Stimulation pads (reference 3 in Figure 1) are typically placed over the calf muscles.
To avoid muscle atrophy a typical stimulation pattern implemented from stimulation device 4 is:
∘ Frequency: 30-50 Hz;
∘ Pulse characteristics: 300-500-µs-long biphasic rectangular pulses;
∘ On/off ratio: 5-10s/10 s with a ramp-up of 0.5-2 s and a ramp-down of 0.5-2 s
∘ Duration: 5-20 minutes repeated 5-10 times during the day.

### Low intensity stimulation (Edema & DVT &wounds)

∘ Typically the intensity threshold is set at approximately 2 times the threshold that mediate minimal muscle contraction with typically varying frequencies between (range, 20―50 Hz), durations (range, 4―9 seconds).
∘ Stimulation pads (2-4) are typically placed over the calf muscles
∘ The duration is typically between 2-12h/day, but can be applied 24/7 if needed

### Example 3

Optimization of silicone: graphene ratio.

To optimize impedance while maintaining enough flexibility the ratio between silicone and graphene has been optimized.

Figure 3 shows that impedance below 100 is reached at a concentration of graphene of 25%.

At a concentration of 27% a preferable stiffness is obtained comparable to that of the skin which is from 0.3 to 0.9 MPa (data not shown) while maintaining an impedance around acceptable 300 is obtained.

### Example 4

Effect of hydrophobic layer on top of stimulation pad.

To obtain additional control of the local environment over the skin, we added a polytetrafluoroethylene (PTFE) membrane and investigated how humidity can be regulated. Figure 4 shows how water evaporate from the following surfaces:
- Uncovered surface
- Silicone mesh without PTFE impregnation
- Silicone mesh with one layer of PTFE impregnation
- Silicone mesh with 10 layers of PTFE impregnation

Figure 4 shows that the evaporation rate can be controlled by changing the layer above the stimulation pad. In sum the distance, shown as D in Figure 2, between the silicone threads, the diameter of the threads, the integration into the woven textile, and the presence of PTFE all influences the overall permeability.

### Example 5

### Effect of integrated electrode pads on skin - Skin tolerance test (7 days) Aim

To test the effect of using an electrode pad which is permeable due to its microporosity of the silicone threads, and thereby avoid skin irritation. In a qualitative test patches were attached to the skin of the lower limb of three subjects and monitored daily by each subject that rated any type of discomfort (rash, pain, itch). Photos of the skin were taken daily.

### Materials

In the used protocol two electrodes made from medical grade silicone doped with graphene is placed, one at each lower leg for 7 days on the test subjects. Prior to their placement, the skin is shaved, the area marked with water proof ink, and the area is photographed. The pads are kept in place by applying a T.E.D. stocking. For every 24 hours the pads are shortly removed in order to photograph the area. Manufacturing of the pads are further described in example 7.

These pads used the micro structures with a strand thickness of 1 and 2 mm, respectively. Pores were 2 and 5 mm in width. The results demonstrate sufficient breathability using these configurations.

### Results

The accumulated results of a skin tolerance test from 3 subjects are presented in table 1.

**Table 1:**

| Day | Gener (VAS 0-10) | | | | | |
|---|---|---|---|---|---|---|
| | Rash | | Pain | | Itch | |
| | Right | Left | Right | Left | Right | Left |
| 0 | 0 | 0 | 0 | 0 | 0 | 0 |
| 1 | 0 | 0 | 0 | 0 | 0 | 0 |
| 2 | 0 | 0 | 0 | 0 | 0 | 0 |
| 3 | 0 | 0 | 0 | 0 | 0 | 0 |
| 4 | 0 | 0 | 0 | 0 | 0 | 0 |
| 5 | 0 | 0 | 0 | 0 | 0 | 0 |
| 6 | 0 | 0 | 0 | 0 | 0 | 0 |
| 7 | 0 | 0 | 0 | 0 | 0 | 0 |

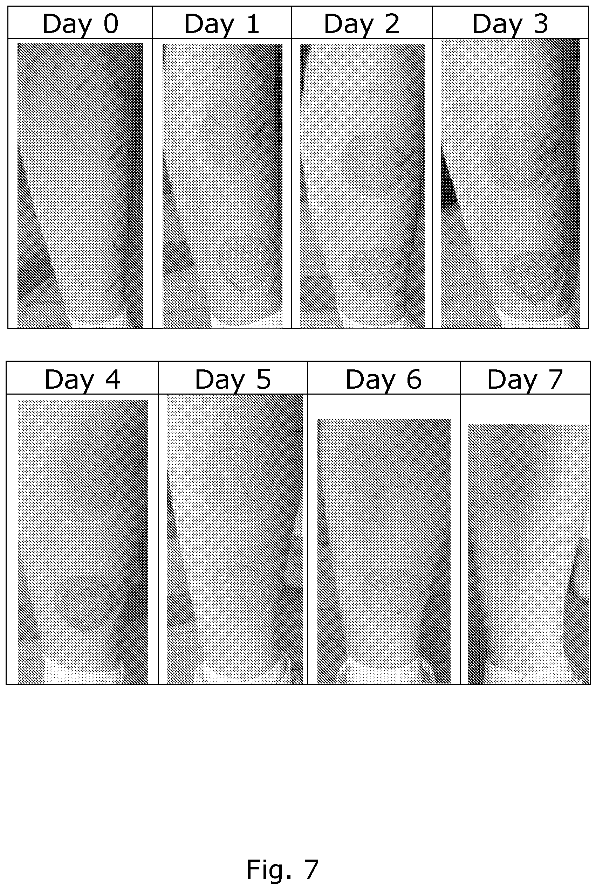

Photos of the right lower limb of one subject each day during the 7 day test period were taken (Figure 7). None of the three subjects experienced any discomfort, rash or pain during the 7 days.

Additionally, testing of a fully commercially available closed pad was performed. This product lead to skin irritation.

Additionally, gel was added to one pad to improve conductivity. This also led to skin irritation.

### Conclusion

None of the three subjects experienced any discomfort, rash or pain during the 7 days. This was on contrast to commercially available closed pad and pads with gel.

### Example 6

A latex - graphene composite pad has also tested with success with respect to its manufacturability and electrical properties. Because of the potential intolerance to latex silicone is still preferred.

### Example 7

Preferred method for producing integrated electrode pads.

### Methods used to manufacture pads as used in example 5.

In the experiment RTV-11 silicone mixed with graphite powder were printed on a modified 3Drag Mendel printer. The following materials were used:
- Two-component silicone RTV-11 white from TECHSIL including DBT (dibutyl tin dilaurate)
- Cross linker from TECHSIL
- Graphite powder from S. Frederiksen A/S
- Grafen nanoplatelets from Sigma-Aldrich
- Marine silicone white from Biltema Danmark A/S
- PRIMA SELECT PLA 1,75 mm black from PrimaFilaments, Sweden
- Syringes 60 mL from KRUUSE
- Transfer pipettes of 2 mL from Sarstedt AG & Co.
- Universal yellow pipettes 2-200 µL from VWR
- 3D printer from Futura Elettronica, Italien Model: 3Drag Mendel Version 1.2
- 3D printer from Open Source Prusa i3 steell, model: AUP3steel
- Ekstruder from Futura Eletronica, Italien Model: choco 3Drag Cod.: 3DCHOCO

The optimum ratio of graphite, graphene, and silicone was determined. Mixtures of 23, 29 and 33 volume percent graphite were made respectively. Mixtures of 5, 9, 17 and 23 volume percent of graphene were made. The mixtures were printed in petri dishes so that they had the same thicknesses. After curing, resistance was obtained in the different pads by measuring with a multimeter.

The preparation of mixtures for printing was conducted in the following way. A plastic cup was weighed. With a 20 mL syringe silicone was transferred to the cup. The weight of the cup including the silicone was noted and the amount of graphite to be applied was calculated from the density of the silicone and graphite. Graphite was weighed and this weight was noted. Prior to addition of graphite to the silicone, a drop of DBT was transferred to the silicone followed by stirring with a spatula. The graphite was then added and thoroughly stirred to the white silicone color and the loose graphite was gone. When the mixture was homogeneous, it was transferred to a 60 mL disposable syringe and ready for printing.

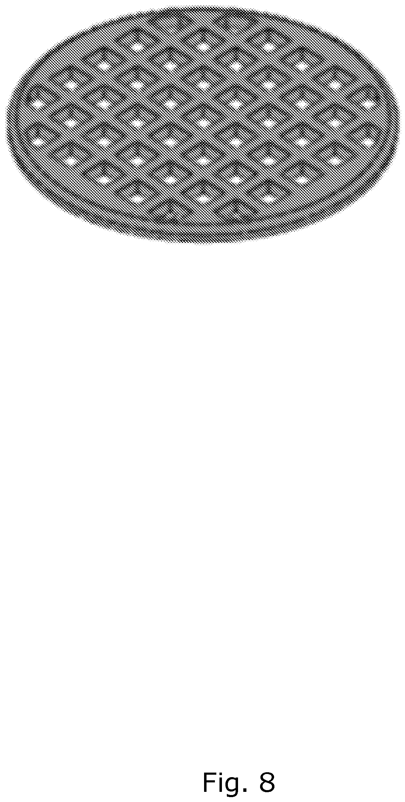

For 3D printing a 3Drag Mendel Version 1.2 was used. The printing head was modified to hold a 60 mL syringe. This syringe was drilled with a 6 mm drill to remove the "luer caps" at the end. Leaving a larger hole in the bottom. A universal pipette tip was then inserted from the inside of the syringe to seal around the piping area and secure a fixed needle position. The graphite-silicone (or graphene-silicone) blend syringe was inserted into the holder, after which the Z-axis of the printer was manually calibrated according to the procedure described by the manufacturer. The following settings for the printer was used and the geometry in figure 8 was printed.
- Nozzle diameter: *1*.*1 mm*
- Extruder temperature: *350 C*
- Print plane temperature: *300 C*
- Min/max print velocity: *8 mm*/*s*, *16 mm*/*s*
- Min/max print plane velocity: *10 mm*/*s*, *20 mm*/*s*
- Filament flow: *300%*
- Lag height: *0*.*8 mm* (first layer height: *0*.*7 mm*)

In an alternative approach, molds were used to prepared pads. The inverse geometry to the one shown in figure 8 was synthesized as a new mold geometry.

Molds were prepared by standard 3D printing using PLA. The following settings of the printer was used:
Nozzle diameter: *0*.*4 mm*
Extruder temperature: *2000 C*
Print plane temperature: *600 C*
Min/max print velocity: *18 mm*/*s*, *40 mm*/*s*
Min/max print plane velocity: *18 mm*/*s*, *60 mm*/*s*
Filament flow: *100%*
Layer height: *0*.*2 mm* (first layer height: *0*.*2 mm*)

Molds were put together two and two with tape, so the bottom and the pattern on the top was aligned. Subsequently, they were lubricated with oil to get the patch out of the mold. Graphite silicone (or graphene-silicone) mixtures with 24.5 volume graphite/graphene were prepared as previously described. This mixture was transferred to the molds, and distributed until the entire mold was covered.

## Claims

1. A compression garment (2) for providing neuromuscular electrical stimulation (NMES), the compression garment comprising:
• at least two integrated electrode pads (3) adapted to provide NMES, wherein the electrode pads comprise silicone polymers, the silicone polymer further comprises graphene, the at least two electrode pads being adapted to be in contact with the skin of a subject when in use; and
• connection means for electrically connecting the at least two integrated electrode pads (3) to an associated stimulation device (4), the stimulation device being adapted to implement one, or more, neuromuscular electrical stimulation patterns through the integrated electrode pads (3) when in contact with the skin,
**characterized in that** the silicone polymers comprise
- 10-40% (w/w) graphene; and
- 60-90% (w/w) silicone.

2. The compression garment (2), according to claim 1, comprising
- 20-35% graphene; and
- 65-80% silicone.

3. The compression garment (2), according to claim 1 or 2, comprising
- 23-32% graphene; and
- 68-77% silicone.

4. The compression garment according to any of the preceding claims, wherein the graphene has an average particle size below 10 microns.

5. The compression garment according to any of the preceding claims, wherein the silicone polymers comprising graphene have an electrical impedance of less than 300.

6. The compression garment according to any of the preceding claims, wherein the integrated electrode pads (3) comprise silicone polymer strands positioned with an average distance between the individual polymer strands of 0.15 mm to 5 mm.

7. The compression garment according to any of the preceding claims, wherein the compression garment with the silicone polymers comprising graphene has a stiffness in the range 0.05 to 0.3 MPa.

8. The compression garment according to any of the preceding claims, wherein the at least two integrated electrode pads are woven into the garment constituting the compression garment.

9. The compression garment according to any of the preceding claims, wherein the part of the electrodes adapted to be in contact with the skin of the subject is further covered with one or more, preferably hydrophobic, cover layers (5) on a side adapted to face away from the skin, to maintain moisture at the skin-electrode interface.

10. The compression garment according to any of the preceding claims, wherein the compression garment comprises polyamide polytetrafluoroethylene (PTFE), polyurethanes, polyethylenes, polypropylenes, polyethylene terephelate, and/or polyesters, fabrics and/or elastan.

11. The compression garment according to any of the preceding claims, wherein the integrated electrode pads are adapted to provide an electrical stimulation with peak currents up to 500mA.

12. A neuromuscular electrical stimulation system (1) comprising
• a compression garment (2) with at least two integrated electrode pads (3) adapted to provide neuromuscular electrical stimulation (NMES), wherein the at least two integrated electrode pads (3) comprise silicone polymers, the silicone polymer further comprises graphene, the at least two electrode pads being adapted to be in contact with the skin of a subject when in use; and
• a stimulation device (4) electrically connected to the at least two integrated electrode pads (3) in the compression stocking (2), adapted to implement one, or more, neuromuscular electrical stimulation patterns through the integrated electrode pads (3),
**characterized in that** the silicone polymers comprise
- 10-40% (w/w) graphene; and
- 60-90% (w/w) silicone.

13. The system according to claim 1, wherein the system further comprises a blood occlusion device (7) adapted to be positioned on a subject to reduce the blood flow to and/or from the muscle during at least part of the neuromuscular electrical stimulation.

14. The system according to claim 13, wherein the blood occlusion device is adapted to provide a functional compression pressure in the range 50-150 mmHg.

15. The system according to claim 13 or 14, wherein the blood occlusion device is a pneumatic cuff.

## Patentansprüche

1. Kompressionsbekleidung (2) zum Bereitstellen einer neuromuskulären elektrischen Stimulation (NMES), wobei die Kompressionsbekleidung umfasst:
• mindestens zwei integrierte Elektrodenpads (3), die dazu angepasst sind, NMES bereitzustellen, wobei die Elektrodenpads Silikonpolymere umfassen, das Silikonpolymer weiter Graphen umfasst, wobei die mindestens zwei Elektrodenpads dazu angepasst sind, bei Verwendung mit der Haut eines Subjekts in Kontakt zu stehen; und
• Verbindungsmittel zum elektrischen Verbinden der mindestens zwei integrierten Elektrodenpads (3) mit einer zugeordneten Stimulationsvorrichtung (4), wobei die Stimulationsvorrichtung dazu angepasst ist, bei Kontakt mit der Haut ein oder mehrere neuromuskuläre elektrische Stimulationsmuster durch die integrierten Elektrodenpads (3) zu implementieren,
**dadurch gekennzeichnet, dass** die Silikonpolymere umfassen
- 10-40 Gew.-% Graphen; und
- 60-90 Gew.-% Silikon.

2. Kompressionsbekleidung (2) nach Anspruch 1, umfassend
- 20-35 % Graphen; und
- 65-80 % Silikon.

3. Kompressionsbekleidung (2) nach Anspruch 1 oder 2, umfassend
- 23-32 % Graphen; und
- 68-77 % Silikon.

4. Kompressionsbekleidung nach einem der vorstehenden Ansprüche, wobei das Graphen eine durchschnittliche Partikelgröße von unter 10 Mikrometer aufweist.

5. Kompressionsbekleidung nach einem der vorstehenden Ansprüche, wobei die Graphen umfassenden Silikonpolymere eine elektrische Impedanz von weniger als 300 aufweisen.

6. Kompressionsbekleidung nach einem der vorstehenden Ansprüche, wobei die integrierten Elektrodenpads (3) Silikonpolymerstränge umfassen, die mit einem durchschnittlichen Abstand zwischen den einzelnen Polymersträngen von 0,15 mm bis 5 mm positioniert sind.

7. Kompressionsbekleidung nach einem der vorstehenden Ansprüche, wobei die Kompressionsbekleidung mit den Graphen umfassenden Silikonpolymeren eine Steifigkeit im Bereich von 0,05 bis 0,3 MPa aufweist.

8. Kompressionsbekleidung nach einem der vorstehenden Ansprüche, wobei die mindestens zwei integrierten Elektrodenpads in die die Kompressionsbekleidung bildende Bekleidung eingebunden sind.

9. Kompressionsbekleidung nach einem der vorstehenden Ansprüche, wobei der Teil der Elektroden, der dazu angepasst ist, mit der Haut des Subjekts in Kontakt zu stehen, weiter an einer Seite mit einer oder mehreren, vorzugsweise hydrophoben, Deckschichten (5) bedeckt ist, die dazu angepasst ist, von der Haut abgewandt zu sein, um Feuchtigkeit an der Haut-Elektroden-Grenzfläche beizubehalten.

10. Kompressionsbekleidung nach einem der vorstehenden Ansprüche, wobei die Kompressionsbekleidung Polyamid, Polytetrafluorethylen (PTFE), Polyurethane, Polyethylene, Polypropylene, Polyethylenterephthalat, und/oder Polyester, Gewebe und/oder Elastan umfasst.

11. Kompressionsbekleidung nach einem der vorstehenden Ansprüche, wobei die integrierten Elektrodenpads dazu angepasst sind, eine elektrische Stimulation mit Spitzenströmen von bis zu 500 mA bereitzustellen.

12. Neuromuskuläres elektrisches Stimulationssystem (1) umfassend
• eine Kompressionsbekleidung mit mindestens zwei integrierten Elektrodenpads (3), die dazu angepasst sind, eine neuromuskuläre elektrische Stimulation (NMES) bereitzustellen, wobei die mindestens zwei integrierten Elektrodenpads (3) Silikonpolymere umfassen, das Silikonpolymer weiter Graphen umfasst, wobei die mindestens zwei Elektrodenpads dazu angepasst sind, bei Verwendung mit der Haut eines Subjekts in Kontakt zu stehen; und
• eine Stimulationsvorrichtung (4), die mit den mindestens zwei integrierten Elektrodenpads (3) in dem Kompressionsstrumpf (2) elektrisch verbunden ist, dazu angepasst, ein oder mehrere neuromuskuläre elektrische Stimulationsmuster durch die integrierten Elektrodenpads (3) zu implementieren,
**dadurch gekennzeichnet, dass** die Silikonpolymere umfassen
- 10-40 Gew.-% Graphen; und
- 60-90 Gew.-% Silikon.

13. System nach Anspruch 1, wobei das System weiter eine Blutokklusionsvorrichtung (7) umfasst, die dazu angepasst ist, an einem Subjekt positioniert zu werden, um den Blutfluss zu und/oder von dem Muskel während zumindest eines Teils der neuromuskulären elektrischen Stimulation zu verringern.

14. System nach Anspruch 13, wobei die Blutokklusionsvorrichtung dazu angepasst ist, einen funktionellen Kompressionsdruck im Bereich von 50-150 mmHg bereitzustellen.

15. System nach Anspruch 13 oder 14, wobei die Blutokklusionsvorrichtung eine pneumatische Manschette ist.

## Revendications

1. Vêtement de compression (2) destiné à la stimulation électrique neuromusculaire (NMES), le vêtement de compression comprenant:
• au moins deux coussinets (3) à électrodes intégrées, conçus pour fournir une NMES, les coussinets à électrodes comprenant des polymères silicone, les polymères silicone comprenant en outre du graphène, lesdits au moins deux coussinets à électrodes étant conçus pour être en contact avec la peau d'un sujet lors de l'utilisation ; et
• un moyen de connexion destiné à connecter électriquement les au moins deux coussinets (3) à électrodes intégrées à un dispositif de stimulation (4) associé, le dispositif de stimulation étant conçu pour exécuter un, ou plusieurs, modes de stimulation électrique neuromusculaire à travers les coussinets (3) à électrodes intégrées, lors du contact avec la peau,
**caractérisé en ce que** les polymères silicone comprennent
- 10-40% (p/p) de graphène ; et
- 60-90% (p/p) de silicone.

2. Vêtement de compression (2), selon la revendication 1, comprenant
- 20-35% de graphène ; et
- 65-80% de silicone.

3. Vêtement de compression (2), selon la revendication 1 ou 2, comprenant
- 23-32% de graphène ; et
- 68-77% de silicone.

4. Vêtement de compression selon l'une quelconque des revendications précédentes, dans lequel le graphène a une taille moyenne de particule inférieure à 10 microns.

5. Vêtement de compression selon l'une quelconque des revendications précédentes, dans lequel les polymères silicone comprenant du graphène ont une impédance électrique de moins de 300.

6. Vêtement de compression selon l'une quelconque des revendications précédentes, dans lequel les coussinets (3) à électrodes intégrées comprennent des brins de polymère silicone disposés à une distance moyenne entre les brins de polymère individuels de 0,15 mm à 5 mm.

7. Vêtement de compression selon l'une quelconque des revendications précédentes, dans lequel le vêtement de compression comportant les polymères silicone comprenant du graphène a une rigidité dans la plage de 0,05 à 0,3 MPa.

8. Vêtement de compression selon l'une quelconque des revendications précédentes, dans lequel les au moins deux coussinets à électrodes intégrées sont tissés dans le vêtement constituant le vêtement de compression.

9. Vêtement de compression selon l'une quelconque des revendications précédentes, dans lequel la partie des électrodes conçue pour être en contact avec la peau du sujet est en outre couverte d'une ou plusieurs couches de recouvrement (5), de préférence hydrophobes, sur un côté conçu pour être opposé à la peau, afin de maintenir l'humidité à l'interface peau-électrode.

10. Vêtement de compression selon l'une quelconque des revendications précédentes, dans lequel le vêtement de compression comprend du polyamide, du polytétrafluoroéthylène (PTFE), des polyuréthanes, des polyéthylènes, des polypropylènes, du poly(éthylène téréphtalate), et/ou des polyesters, des tissus et/ou de l'élasthanne.

11. Vêtement de compression selon l'une quelconque des revendications précédentes, dans lequel les coussinets à électrodes intégrées sont conçus pour fournir une stimulation électrique avec des courants de crête de jusqu'à 500 mA.

12. Système de stimulation électrique neuromusculaire (1) comprenant
• un vêtement de compression comportant au moins deux coussinets (3) à électrodes intégrées, conçus pour fournir une stimulation électrique neuromusculaire (NMES), lesdits au moins deux coussinets (3) à électrodes intégrées comprenant des polymères silicone, les polymères silicone comprenant en outre du graphène, lesdits au moins deux coussinets à électrodes étant conçus pour être en contact avec la peau d'un sujet lors de l'utilisation ; et
• un dispositif de stimulation (4) connecté électriquement auxdits au moins deux coussinets (3) à électrodes intégrées dans le bas de compression (2), conçu pour exécuter un, ou plusieurs, modes de stimulation électrique neuromusculaire à travers les coussinets (3) à électrodes intégrées,
**caractérisé en ce que** les polymères silicone comprennent
- 10-40% (p/p) de graphène ; et
- 60-90% (p/p) de silicone.

13. Système selon la revendication 1, dans lequel le système comprend en outre un dispositif d'occlusion de sang (7) conçu pour être placé sur un sujet afin de réduire le flux sanguin vers et/ou depuis le muscle pendant au moins une partie de la stimulation électrique neuromusculaire.

14. Système selon la revendication 13, dans lequel le dispositif d'occlusion de sang est conçu pour fournir une pression de compression fonctionnelle dans la plage de 50-150 mm Hg.

15. Système selon la revendication 13 ou 14, dans lequel le dispositif d'occlusion de sang est un manchon pneumatique.
